# EUROPEAN PATENT APPLICATION

(11) **EP 4 696 317 A1**
(43) Date of publication of application: **18.02.2026**
(21) Application number: 24788107.1
(22) Date of filing: 10.04.2024
(51) Int. Cl.: A61K 38/17, A61K 38/18, A61P 17/02, A61P 3/10

(54) **DRUG FOR REPAIRING REFRACTORY WOUND**

(30) Priority: 12.04.2023 CN 202310388253; 25.10.2023 CN 202311392902
(71) Applicant: Shanghai Celleaf Biotechnology Co., Ltd., Shanghai 200241 (CN)
(72) Inventor: ZHANG, Wenjie, Shanghai 200241 (CN); YANG, Tingjia, Shanghai 200241 (CN)
(74) Representative: Mewburn Ellis LLP
(86) International application number: PCT/CN2024/087035
(87) International publication number: WO 2024/213005

(57) **Abstract**

A drug for repairing a refractory wound. Provided is the use of annexin in the preparation of a composition or a preparation for repairing a refractory wound.

## Description

### Technical field

The present invention relates to the field of biomedicine, specifically to a drug for repairing refractory wounds.

### Background

Wound repair refers to a series of pathological and physiological processes in which local tissues are repaired and restored through regeneration and reconstruction after the skin and other tissues are ruptured or damaged by trauma or other causes. The process of wound repair can be roughly divided into three stages: inflammatory response, proliferation, and remodeling. These three stages occur sequentially and overlap with each other. When one stage goes out of control, it can lead to tissue damage that cannot be repaired normally, forming refractory wounds. Refractory wounds have complex pathogenesis, a long course, involve multiple disciplines, are difficult to treat, and are high in the cost of treatment,, which brings heavy physical and mental pressure and economic burden to the subjects.

Refractory wounds associated with diabetic foot disease are relatively common in clinical practice, and also very difficult to treat, and the treatment time is long and the cost is high, involving multiple disciplines. The abnormal repair of chronic refractory diabetic wounds is mainly manifested by decreased neovascularization and collagen deposition, prolonged inflammatory response time, and enhanced oxidative stress. In diabetic wounds, the inflammatory response at the early stage of healing appears later and lasts longer than that in normal wounds. The high glucose and high-fat environment in the wound area can lead to a decrease in the chemotaxis and migration function of peripheral blood neutrophils, resulting in a delay in the entry of neutrophils into the wound during the early stages of inflammation. At the same time, the accumulated advanced glycation end products in the wound bind to neutrophils, interfering with the normal interaction between inflammatory cells and vascular endothelial cells. In addition, glucose and its metabolites in diabetic wounds can lead to excessive production of reactive oxygen species through DAG-PKC signal transduction pathway, polyol pathway, non-enzymatic glycosylation reaction and other pathways. At the same time, neutrophils and macrophages during the inflammatory response phase can also generate a large amount of reactive oxygen species under the stimulation of factors such as high glucose and high fat. Reactive oxygen species not only act as second messengers for certain cytokines in the cells, inducing apoptosis or necrosis, but also promote the release of inflammatory factors, leading the wound to enter a vicious cycle of inflammation-oxidative stress-inflammation.

At present, most research at home and abroad focus on the role of growth factors in the healing of damaged wounds, which can accelerate the healing of refractory wounds cause by conditions such as diabetes, malnutrition, infection and the use of steroids, chemotherapy drugs and radiotherapy. However, the human body is a complex organism, and the growth factors needed for wounds healing do not exist in isolation, but rather interact with each other. Therefore, there is a need in this field to provide more products for repairing refractory wounds.

### Summary of the invention

The purpose of the present invention is to provide a drug for repairing refractory wounds (especially those caused by diabetes).

The present invention provided a use of annexin in the preparation of pharmaceutical compositions for repairing refractory wounds.

In another preferred embodiment, the annexin is annexin A, more preferably annexin A5.

In another preferred embodiment, the refractory wound refers to a wound that cannot be repaired in a normal, orderly and timely manner to achieve an anatomically and functionally complete state.

In another preferred embodiment, the refractory wounds are chronic refractory wounds caused by traumatic ulcers.

In another preferred embodiment, the refractory wounds are chronic refractory wounds caused by pressure ulcers (such as pressure sores).

In another preferred embodiment, the refractory wounds are refractory wounds caused by arterial ulcers (such as diabetic ulcers).

In another preferred embodiment, the repairing refractory wounds includes improving wound healing rate and/or shortening wound healing time.

In another preferred embodiment, the pharmaceutical composition comprises other wound healing drugs; Preferably, the other wound healing drugs are selected from the group consisting of: recombinant human epidermal growth factor (rhEGF), recombinant bovine basic fibroblast growth factor (rhbFGF), or a combination thereof.

In another preferred embodiment, the annexin is derived from humans, mice, or rats.

In another preferred embodiment, the composition or preparation further comprises a pharmaceutically acceptable salt.

In another preferred embodiment, the dosage form of the composition or preparation is selected from the group consisting of: liquid dosage form and solid dosage form.

In another preferred embodiment, the dosage form of the composition or preparation is selected from the group consisting of: tablets, capsules, powders, granules, lozenges, patches, suppositories, pills, pastes, oral liquids, freeze-dried powder injections, microcapsules, dressings, gels, liniments or injections.

The present invention also provided a method for repairing refractory wounds, comprising a step of: administering a pharmaceutical composition comprising the annexin to the wounds of a subject.

In another preferred embodiment, the subject is a human or a non-human mammal (such as a monkey, a rat, or a mouse).

It should be understood that within the scope of the present invention, the above-mentioned technical features of the present invention and the technical features specifically described in the following (such as embodiments) can be combined with each other to form a new or preferred technical solution. Limited to space, it will not be repeated here.

### Description of the drawings

Figure 1: Panel A. Control group, model control group, and treatment group were photographed at day 0, 3, 7, 10, and 14, respectively; Panel B. Day 0, 3, 7, 10, and 14 after wound healing, the percentage of wound area in each group compared to the original wound area.
Figure 2: Panel A. On the 14th day, H&E staining was performed on the wound area; Panel B. On the 14th day, MASSON trichrome staining (collagen deposition stained in blue) was performed on the wound area.
Figure 3: Panel A. Immunohistochemical staining of wound tissue; Panel B. Quantification of CD68+cell density in each group; Panel C. Statistical analysis of CD31+neovascularization in each group; Panel D. Statistical analysis of PCDN in each group.

### Detailed Description of the invention

After extensive and in-depth research, the inventor found through extensive screening and testing that annexin A5 can reduce the number of neutrophils during the repair process of refractory wounds, alleviate the dense inflammatory zone formed by the scattering of a large number of neutrophils around the wound, and at the same time, reduce the release of a large number of inflammatory factors due to the binding of neutrophils with advanced glycation end products outside the vascular tissue. These inflammatory factors can exacerbate the inflammatory response and oxidative stress of neutrophils. Therefore, annexin A5 can significantly improve the treatment of refractory wounds, increase wound healing rate, and shorten wound healing time, making it very suitable for the preparation of drugs for repairing refractory wounds. The present invention has been accomplished on this basis.

### Terms

Unless otherwise defined, all technical and scientific terms used herein have the same meaning as would normally be understood by those of ordinary skill in the art to which the present invention belongs.

As used herein, when used in reference to a specific enumerated value, the term "about" means that the value can vary from the enumerated value by no more than 1%. For example, as used herein, the expression "about 100" includes all values between 99 and 101 (e.g., 99.1, 99.2, 99.3, 99.4, etc.).

As used herein, the terms "contain" or "include (comprise)" may be open-ended, semi-closed, and closed-ended. In other words, the term also includes "consisting essentially of", or "consisting of".

Unless otherwise specified, the term "room temperature" or "normal temperature" refers to a temperature of 4-40 °C, preferably 25±5 °C.

### Active ingredient

Annexin are a widely distributed class of calcium dependent phospholipid binding proteins. Based on molecular phylogenetics and comparative genomics analysis, this protein family is uniformly named annexin (abbreviated as Anx), followed by capital letters representing different species: A, B, C, D, E, representing vertebrates, invertebrates, fungi and slime molds, plants, and protists, respectively. As of now, the Anx A family has a total of 12 members: A1-A11 and A13 (A12 not specified).

Representatively, the annexin mentioned in the present invention is Anx A. More preferably, the annexin comprises annexin A5. Annexin A5 (AnxA5) is widely distributed in various tissues and cells of the body. In vitro studies have found that AnxA5 has multiple functions such as anti-phospholipase, anticoagulant, and anti-kinase. However, its specific in vivo functions are not very clear, and it is currently mainly used as a reagent for detecting cell apoptosis.

The annexin that can be used in the present invention are not particularly limited and can be annexin derived from any organism, preferably from mammals (such as primates), and more preferably from humans, monkeys, rats, or mice. The annexin of the present invention can also be its functional analogs, such as a protein that has 50%, 60%, 70%, 75% or more identity with human annexin, such as 80%, 85% or more, 90% or more, or even more preferably 95% or 99% or more identity with human annexin; It should be understood that 'annexin' include wild-type or mutant (including truncated) annexin, as long as the mutant annexin retains or maintains the detoxification activity of wild-type annexin, and it can also be used in the present invention. In addition, annexin can also be multimers, fusion proteins, or chemically modified variants (such as PEG modification) of natural annexin, provided that these variants possess the detoxification activity of the wild-type annexin.

### Pharmaceutical composition and mode of administration

The pharmaceutical composition of the present invention comprises the active ingredients mentioned above, as well as pharmaceutically acceptable carriers.

Experimental results have shown that the active ingredient of the present invention can improve the healing rate of refractory wounds and shorten the healing time, making it highly suitable for the preparation of drugs for repairing refractory wounds.

In the present invention, the "wound" refers to a wound formed on the human skin due to skin damage or loss of skin tissue caused by external factors, and wound repair is also known as wound healing. According to the healing time, wounds can usually be divided into acute wounds and chronic wounds. Acute wounds mainly refer to sudden injuries (such as wounds within 7 days after injury), such as bruise, abrasions, scratches, stabs, etc.; Chronic wounds (i.e. refractory wounds) refer to acute wounds that cannot heal according to the normal physiological mechanism of the body due to various reasons, resulting in prolonged and unhealed wounds. Generally, from a medical perspective, wounds that cannot heal for more than one month are called chronic wounds or refractory wounds. The causes of chronic wounds include (but are not limited to): injuries caused by pressure (such as bedsores), ulcers caused by varicose veins or insufficient arterial blood supply, diabetic ulcers, etc.; or atypical wounds caused by various diseases such as skin and soft tissue infections, vascular inflammation, connective tissue diseases, tumors, etc.; long term radiation exposure can lead to wound tissue necrosis, infection, and wounds with poor prognosis.

Preferably, the wounds are selected from the group consisting of: chronic refractory wounds caused by traumatic ulcers, chronic refractory wounds caused by pressure ulcers (such as pressure sores), and chronic refractory wounds caused by arterial ulcers (such as diabetic ulcers).

In another preferred embodiment, the repairing refractory wounds includes improving the wound healing rate and/or shortening the wound healing time to achieve an anatomical and functional integrity of the wound. For example, compared to not using annexin, wounds treated with annexin have an increased healing rate and/or a shortened healing time by 10%, 20%, 50%, or even 100%; or annexin repair wounds that cannot be repaired by other conventional wound repair drugs.

Pharmaceutically acceptable excipients" and "pharmaceutically acceptable carriers" refer to substances that facilitate the formulation and/or administration of active ingredients and/or their absorption by individuals, and can be included in the disclosed composition without causing significant adverse toxicological effects on the individual.

In some embodiments, the pharmaceutical composition of the present invention may be in solid or liquid form.

The active ingredient can be administered to the subject through any suitable routes, including oral administration, parenteral administration, inhalation of spray, local administration, rectal administration, nasal administration, buccal administration, vaginal administration or via an implantable kit. The term 'parenteral' used herein includes subcutaneous, intravenous, intramuscular, intra-articular, synovial, sternal, intrathecal, intrahepatic, intralesional and intracranial injection or infusion techniques. Preferably, the composition is administered orally, subcutaneously, or intravenously.

The pharmaceutical composition of the present invention suitable for oral administration will typically be in the form of discrete units in solid form, such as tablets, capsules, cachets, granules, lozenges, or pills, or in liquid form, such as liquid formulations, injectable or infusible solutions or suspensions.

Preferably, the pharmaceutical composition of the present invention is suitable for targeted local application to the wound area, and the pharmaceutical composition suitable for local application is typically made of powders, patches, injections, pastes, dressings, gels (such as gels based on gelatin methacryloyl), etc..

The active ingredient of the present invention can be used alone or in combination with other therapeutic agents, which can be selected from the group consisting of: antibacterial agents, anti-inflammatory agents, other wound healing drugs (such as recombinant human epidermal growth factor (rhEGF), recombinant bovine basic fibroblast growth factor (rhbFGF)), or combinations thereof.

The precise amount of a compound required to provide a therapeutically effective amount to an individual will depend on the mode of administration, the type and severity of the disease and/or condition, and individual characteristics such as general health status, age, gender, weight, and drug tolerance. Ordinary technicians in this field will be able to determine the appropriate dosage based on these and other factors. When administered in conbination with other therapeutic agents, the 'therapeutically effective amount' of any other therapeutic agent will depend on the type of the medication used. The appropriate dosage for approved therapeutic agents is known and can be adjusted by those skilled in the art based on the individual's condition, the type of disease being treated, and the amount of the compound of the present invention used, such as the dosage reported in literature and recommended in Physician's Desk Reference (57th edition, 2003). Preferably, the composition should be formulated such that a dose of 0.01-100 mg/kg body weight/day of the inhibitor can be administered to patients receiving these compositions. In certain embodiments, the composition of the present invention provides a dose of 0.01 mg to 50 mg. In other embodiments, a dose of 0.1 mg-25 mg or 5 mg-40 mg is provided.

Examples of the subjects of administration of the pharmaceutical composition or therapeutic agents of the present invention include mammals (such as humans, mice, rats, hamsters, rabbits, cats, dogs, cows, sheep, monkeys, etc.).

The present invention also provides a treatment method comprising a step of: administering the active ingredient described in the present invention or administering the pharmaceutical composition described in the present invention to a subject in need of treatment for repairing refractory wounds. Preferably, the pharmaceutical composition is directly administered to the wound area.

### The main advantages of the present invention include:

The present invention provides a use of annexin for repairing refractory wounds, which has significant therapeutic effects, can effectively promote the healing of refractory wounds, improve wound healing rates, shorten wound healing time, and has a simple and economical medication method. Therefore, it is very suitable for the preparation of drugs for the repair of refractory wounds and has important application prospects.

The present invention will be further explained below in conjunction with specific Examples. It should be understood that these embodiments are only used to illustrate the present invention and not to limit the scope of the present invention. In the following examples, the test methods without specific conditions are usually in accordance with conventional conditions or the conditions recommended by the manufacturer. Unless otherwise specified, percentages and parts are calculated by weight.

### Example 1

Experimental animals: male C57BL/KsJdb/db mice (8 weeks, 36-40g) were used to model type II diabetes; annexin A5: extracted and purified from human adipose tissue, accession number: P08758.
1.2 Experimental grouping and administration: Randomly divided into 3 groups, administration and dosage regimen are as follows:

| Group number | Group | Number of animals | Mode of administration | Administration |
|---|---|---|---|---|
| control | normal control group | 5 | subcutaneous injection | PBS buffer solution |
| GELMA | model control group | 5 | subcutaneous injection | GELMA |
| GELMA-A5 | administration group | 5 | subcutaneous injection | GELMA-Annexin A5 (dose: 2mg/mL) |

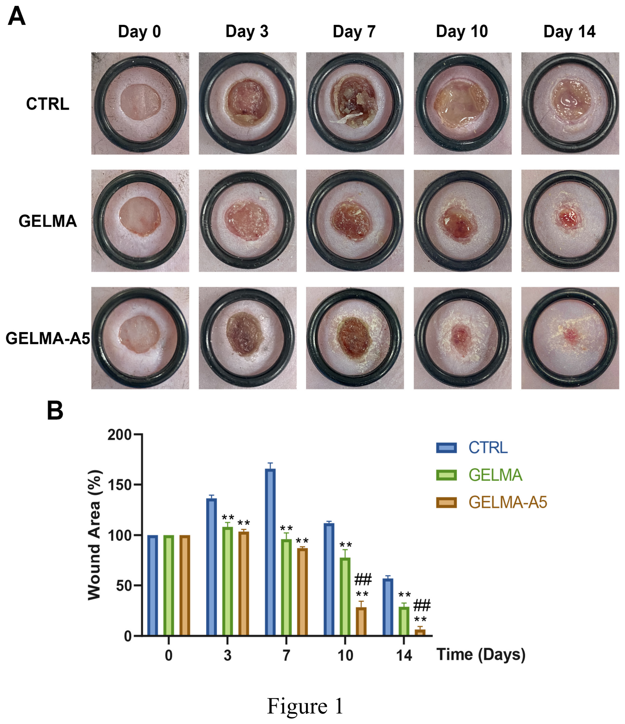

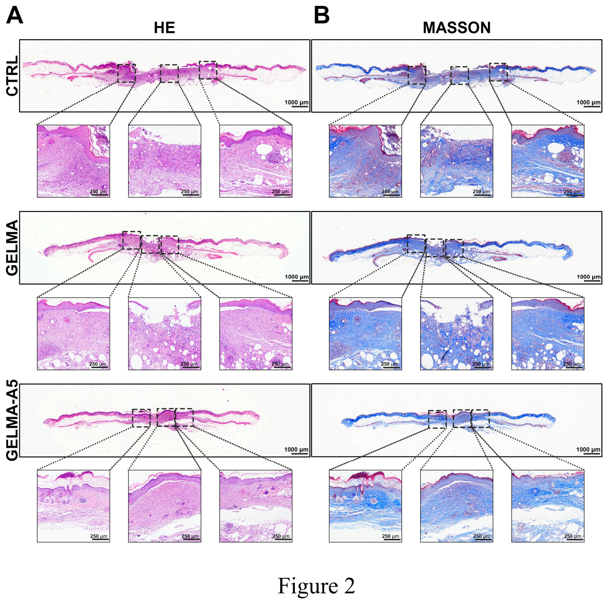

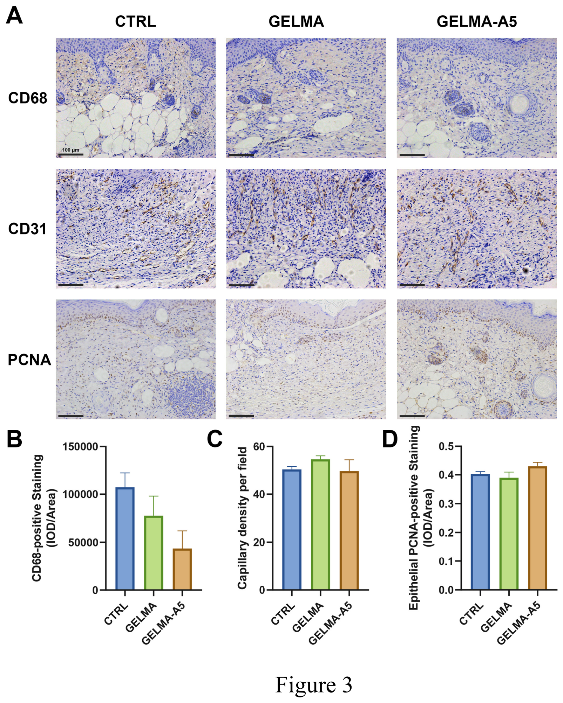

1.3 Establishment of a type II diabetes model in experimental rats: the model control group and the administration group were given a single dose of 70 mg/kg Streptozotocin (STZ) intraperitoneally after fasting for 24 hours. Random blood glucose levels were measured each week by tail vein sampling before and after injection, and the body weight of the induced experimental mice was observed simultaneously. If the blood glucose level before induction is less than 8.9 mmol/L, and the blood glucose level after induction is more than or equal to 11.2 mmol/L, and the body weight decreases significantly, it can be regarded as successful induction of diabetes model.
1.4 Formation method of refractory wounds: round wound with 6 mm diameter was made on the back of type II diabetes experimental rats by ophthalmic scissors;
1.5 Testing indicators
1.5.1 After administration, refractory wounds were photographed at day 0, 3, 7, 10, and 14. The image data was input into a computer and analyzed using Image Pro Plus software version 6.0 (Media Cybernetics, Rockville, MD, USA) to calculate the wound healing rate and time. Wound healing rate: Wound healing rate = (original wound area - terminal wound area)/original wound area x 100%. Wound healing time: The number of days from the time the wound is treated to the day the wound healing rate reaches 90%.
1.5.2 Tissue staining: 14 days later, the wound and the surrounding tissues (0.5 cm) were taken and stained with HE staining to determine the morphology of epidermal and dermal tissues, as well as keratinocytes and fibroblasts. The specific operation can be: the tissue was fixed in 10% formalin solution, embedded in paraffin, sectioned, deparaffinized with xylene, dehydrated through anhydrous ethanol and 90% ethanol, rinsed with water, stained with hematoxylin, differentiated with hydrochloric acid, rinsed again with water, counterstained with eosin, dehydrated through 95% ethanol and anhydrous ethanol, xylene, mounted, baked, and examined under an optical microscope to observe fibroblasts, inflammatory cells, capillaries, and collagen fibers within the dermal granulation tissue.
1.5.3 Immunohistochemical staining: to evaluate the infiltration of inflammatory cells, vascular proliferation, and cell proliferation in the wound, wound tissue sections were examined on the 14th day, and CD68⁺ macrophage protein, CD31⁺, and proliferating cell nuclear antigen (PCNA) were stained.
1.5.4 In the present invention, data statistics are analyzed using statistical software SPSS 18.0. The experimental results are organized as quantitative data and described using mean ± standard error. The results were analyzed using one-way analysis of variance and Tukey's-Test for pairwise comparisons between groups. P<0.05 indicates statistically significant differences.
1.6. Results
1.6.1 After administration, the wound was photographed at day 0, 3, 7, 10, and 14 (Figure 1. Panel A). Compared with the control group, the wound area in the administration group significantly decreased from day 3, and the difference between the administration group and the model group was significant from day 10 (Figure 1), quantitative analysis of wound closure showed that the GELMA-A5 group had the highest wound healing rate.
1.6.2 Tissue staining results: H&E stained wound tissue sections (Figure 2, Panel A) were analyzed under microscope. Histological observation showed that on day 14, the wounds treated in the administration group exhibited complete continuous re-epithelialization, while the control group showed almost no re-epithelialization. Although epithelial cells were present in the wounds of the model group, they were not continuous. The regeneration of dermis is mainly identified by MASSON trichrome staining method for collagen, which is the most abundant fiber component in dermal connective tissue. Compared with the control group, the collagen deposition in the administration group was enhanced (Figure 2, Panel B).
1.6.3 Immunohistochemical staining results: On the 14th day after surgery, CD68+ and CD31+ were stained by immunohistochemistry. The infiltration of CD68+ inflammatory cells in the administration group was significantly lower than that of the other two groups (Figure 3, Panel A and Figure 3, Panel B). Compared with the control group, the vascular density increased in both the model group and the administration group (Figure 3, Panel C), and significant cell proliferation was observed in the administration group (Figure 3, Panel D).
1.7 Discussion: the experiment proves that, compared with the model control group, annexin A5 can significantly improve the healing rate and healing speed of refractory wounds in diabetes mice, shorten the wound healing time, and has no side effects, which suggests that annexin A5 is very suitable for the treatment of refractory wounds.

All documents referred to in the present invention are incorporated by reference herein as if each document is individually incorporated by reference. Further, it should be understood that upon reading the above teaching of the present invention, various modifications or changes may be made to the present invention by those skilled in the art, and those equivalents also fall within the scope defined by the appended claims of the present invention.

## Claims

1. A use of annexin in the preparation of pharmaceutical compositions for repairing refractory wounds.

2. The use according to claim 1, wherein the annexin is annexin A5.

3. The use according to claim 1 or 2, wherein the refractory wounds are chronic refractory wounds caused by traumatic ulcers.

4. The use according to claim 1 or 2, wherein the refractory wounds are chronic refractory wounds caused by pressure ulcers (such as pressure sores).

5. The use according to claim 1 or 2, wherein the refractory wounds are chronic refractory wounds caused by arterial ulcers (such as diabetic ulcers).

6. The use according to claim 1, wherein the repairing refractory wounds includes improving wound healing rate and/or shortening wound healing time.

7. The use according to claim 1, wherein the pharmaceutical composition comprises other wound healing drugs; preferably, the other wound healing drugs are selected from the group consisting of: recombinant human epidermal growth factor (rhEGF), recombinant bovine basic fibroblast growth factor (rhbFGF), or a combination thereof.

8. The use according to claim 1, wherein the annexin is derived from humans, mice, or rats.

9. The use according to claim 1, wherein the composition or preparation further comprises a pharmaceutically acceptable salt.

10. The use according to claim 1, wherein the dosage form of the composition or preparation is selected from the group consisting of: tablets, capsules, powders, granules, lozenges, patches, suppositories pills, pastes, oral liquids, freeze-dried powder injections, microcapsules, dressings, gels, liniments or injections.

11. A method for repairing refractory wounds, comprising a step of: administering an annexin or a pharmaceutical composition comprising the annexin to the refractory wounds of a subject.

12. The method according to claim 11, wherein the annexin is annexin A5.
